# EUROPÄISCHE PATENTANMELDUNG

(11) **EP 1 591 125 A1**
(43) Veröffentlichungstag der Anmeldung: **02.11.2005**
(21) Anmeldenummer: 04009213.2
(22) Anmeldetag: 19.04.2004
(51) Int. Cl.: A61K 35/78, A61P 17/08

(54) **Pharmazeutische Zusammensetzung enthaltend Extrakte aus Flechten und Johanniskraut**

(71) Anmelder: Universitätsklinikum Freiburg, 79106 Freiburg (DE)
(72) Erfinder: Schempp, Christoph, Dr. med. PD, 79103 Freiburg (DE); Blindow, Andrea. Dr. med., 79106 Freiburg (DE); Wilken, Kathrin, Dr. med., 79224 Umkirch (DE)
(74) Vertreter: Keller, Günter

(57) **Zusammenfassung**

Offenbart wird eine pharmazeutische Zusammensetzung, die dadurch gekennzeichnet ist, daß sie 0,01 - 20 Gew.-% eines Extraktes aus Flechten und 0,01 - 20 Gew.-% eines Extraktes aus Johanniskraut enthält. Die Zusammensetzung führt zu einer Stabilisierung gelösten Hyperforins und zu einer Potenzierung des antiinflammatorischen Effektes der Extrakte. Die Kombination eignet sich besonders für die äusserliche und innerliche Behandlung verschiedener Hauterkrankungen, insbesondere Akne.

## Beschreibung

Die vorliegende Erfindung betrifft pharmazeutische Zusammensetzungen, die bei solchen Hauterkrankungen eingesetzt werden können, bei denen verschiedene Bakterien, sowie Pilze zum Entstehen dieser Krankheiten beitragen.

Akne, Rosacea und seborrhoisches Ekzem sind Hauterkrankungen des seborrhoischen Formenkreises. Verschiedene Bakterien, insbesondere Propionibacterium acnes oder Corynebacterien, und Hefepilze, insbesondere Pityrosporon, tragen zum Entstehen dieser Hautkrankheiten bei. Ausserdem sind die Krankheiten des seborrhoischen Formenkreises durch Entzündungen der Haarfollikel und des umgebenden Gewebes gekennzeichnet. Diese Hautkrankheiten beeinträchtigen die Lebensqualität der betroffenen Personen erheblich. Leichte bis mittelschwere Formen werden normalerweise mit verschiedenen Lokaltherapeutika behandelt. Zu den üblichen Behandlungsmitteln gehören Peelings (z.B. Benzoylperoxid, Glykolsäure), Antibiotika (z.B. Erythromycin, Clindamycin, Tetrazyklin) und Vitamin A-Säure-Derivate.

Diese üblichen Wirkstoffe sind aus verschiedenen Gründen problematisch: Die lokale Anwendung der Antibiotika ist zwar wirksam, jedoch können Resistenzen entstehen. Diese sind problematisch, da die Antibiotika auch bei anderen schweren Infektionen eingesetzt werden. Benzoylperoxid führt zu Hautreizungen und bleicht außerdem Wäsche. Vitamin A Säure führt ebenfalls zu ausgeprägten Hautreizungen. Die eingesetzten Substanzen haben keine nennenswerte entzündungshemmende Wirkung.

Akneartige Hauterkrankungen sind häufig sehr problematisch. Da die Hautirritationen vor allem im Gesicht auftreten, kann dies zu schweren psychischen Störungen führen. Es besteht daher ein Bedürfnis nach wirksamen pharmazeutischen Zusammensetzungen, die einerseits die unerwünschten Symptome wirksam und schnell beseitigen und andererseits möglichst geringfügige Nebenwirkungen haben.

Gegenstand der vorliegenden Erfindung sind pharmazeutische Zusammensetzungen, die dadurch gekennzeichnet sind, daß sie 0,01 - 20 Gew.-% eines Extraktes aus Flechten und 0,01 - 20 Gew.-% eines Extraktes aus Johanniskraut enthalten.

Ein wesentlicher Bestandteil der erfindungsgemäßen pharmazeutischen Zusammensetzungen ist ein Extrakt aus Flechten. Flechten (Lichenes) sind eine Symbiose aus Schlauchpilzen (Ascomyceten) und Algen. Die Bartflechten (Gattung Usnea) wachsen epiphytisch auf Bäumen und Sträuchern in Gegenden mit hoher Luftfeuchtigkeit. Bartflechten reagieren empfindlich auf Luftverschmutzung und sind deshalb in vielen Gegenden Mitteleuropas ausgestorben oder selten geworden. Die bevorzugt eingesetzte Usnea barbata kommt in den Alpen und anderen Gebirgen Europas sowie in Nordamerika vor. Die Bartflechten bilden ein graues Geflecht aus dünnen Strähnen. Die Arzneidroge stammt heute vorwiegend aus Osteuropa. Usnea barbata enthält wie das Isländisch Moos (Cetraria islandica) verschiedene Flechtensäuren.

Erfindungsgemäß wird ein Extrakt aus Flechten eingesetzt. Hauptbestandteil der wirksamen Komponenten des Extrakts aus Flechten ist die Usninsäure, die in zwei enantiomeren Formen, nämlich der (+)-9bR-Usninsäure und der (-)-9bS-Usninsäure vorkommt. Man geht davon aus, daß vor allem die (+)-Usninsäure wegen ihrer bioziden Wirkung in der Pharmazie und Kosmetik bevorzugt verwendet wird. Im vorliegenden Fall wird bevorzugt das Racemat oder die (+)-9bR-Usninsäure eingesetzt.

Verfahren zur Isolierung von Usninsäure aus Flechten (Usnea barbata) sind im Stand der Technik bekannt (beispielsweise DE 32 13 095). Bei diesen Extraktionsverfahren wird mit organischen Lösungsmitteln oder Alkohol gearbeitet. Die erfindungsgemäß eingesetzten Extrakte können auf diese bekannte Art und Weise hergestellt werden.

Bevorzugt eingesetzt werden Extrakte aus Usnea barbata, die durch eine Hochdruckextraktion mit CO₂ hergestellt wurden. Vorteil dieser Extraktionsmethode ist, daß keine Lösungsmittelrückstände zurückbleiben. Nach der CO₂-Extraktion kann die Usninsäure zu einer praktisch reinen Form aufgereinigt werden. Die Extrakte werden mit Pflanzenöl und Emulgatoren formuliert und sind käuflich erhältlich (Flavex® Naturextrakte GmbH).

Die erfindungsgemäß eingesetzten Extrakte aus Flechten enthalten 2 - 7, bevorzugt 3 - 5 und besonders bevorzugt etwa 4 Gew.-% Usninsäure. In dem CO₂-Extrakt liegen auch Derivate der Usninsäure als weitere Flechtensäure in einer Menge von bis zu 0,5 Gew.-%, bevorzugt bis zu 0,2 Gew.-% und besonders bevorzugt bis zu 0,1 Gew.-% vor.

Der bevorzugt verwendete Bartflechten-CO₂-Extrakt enthält etwa 4% Usninsäure, ein lipophiles Dibenzofuranderivat. Obwohl Allergien gegen Flechtensäuren beschrieben sind, gilt die sensibilisierende Potenz der Flechten als gering und wird durch andere Flechtensäuren als Usninsäure bedingt. Daher werden bevorzugt solche CO₂-Extrakte eingesetzt, die höchstens 0,1 Gew.-% oder besser noch weniger als 0,1 Gew.-% an solchen Flechtensäuren enthalten.

In der Literatur wird beschrieben, dass Usnea-Arten und Usninsäure ein breites antibakterielles Spektrum aufweisen, und dass sie sich deshalb zur Behandlung von Oberflächeninfektionen und Hautulcera eignen. Die Übersicht von Cocchietto M et al. (2002, Naturwissenschaften 89: 137-146) fasst die Erkenntnisse zu den antibakteriellen Wirkungen von Usninsäure zusammen.

Der andere, wesentliche Bestandteil der erfindungsgemäßen pharmazeutischen Zusammensetzungen ist ein Extrakt aus Johanniskraut. Das Johanniskraut (Hypericum perforatum) wird in großem Umfang und seit langem in der Phytomedizin verwendet. Ein wesentlicher Bestandteil des Johanniskrauts ist Hyperforin.

Hyperforin ist ein antibakterieller Inhaltsstoff aus dem Johanniskraut (Hypericum perforatum), der zusätzlich proliferationshemmende Wirkungen aufweist und sich zur Behandlung von Hautkrankheiten eignet (EP 1 131 063). Dieser Inhaltsstoff ist aufgrund seiner ausgeprägten Lipophilie nur in apolaren Lösungsmitteln und Fetten gut löslich. Bei Emulgation in wässrigen Lösungen tritt ein rascher Abbau des Hyperforins auf. Dieses Problem kann umgangen werden, indem verschiedene Salze des Hyperforins hergestellt werden. In dieser Form kann Hyperforin stabil gelagert werden (WO 99/41220). Werden jedoch die Hyperforinsalze in eine wässrige Lösung gebracht, dissoziiert freies Hyperforin und wird ebenfalls rasch abgebaut.

Die pharmakologisch wirksamen Stoffe können aus den geeigneten Pflanzenteilen (u.a. Blüten) des Johanniskrauts mit organischen Lösungsmitteln extrahiert werden. Erfindungsgemäß wird ein Extrakt aus Johanniskraut eingesetzt, wobei es sich bevorzugt um einen CO₂-Extrakt handelt. Aus Johanniskraut (Hypericum perforatum), insbesondere den Zweigspitzen mit Blüten, wird durch Hochdruckextraktion mit CO₂ ein Extrakt hergestellt, der in Pflanzenöl gelöst und mit α-Tocopherol stabilisiert wird. Derartige Extrakte sind kommerziell erhältlich (beispielsweise von Flavex® Naturextrakte GmbH).

Die erfindungsgemäß eingesetzten Extrakte enthalten 5 - 15, bevorzugt 9 - 12 und besonders bevorzugt 10 - 11 % Hyperforin. Der Gehalt an Adhyperforin beträgt bis zu 2 Gew.-%, bevorzugt bis zu 1,5 Gew.-% und besonders bevorzugt bis zu 1,1 Gew.%. In bevorzugter Ausführungsform enthält der erfindungsgemäß eingesetzte Johanniskrautextrakt kein nachweisbares Hypericin. Bei Anwendung der HPLC mit Standardbedingungen für analytischen Nachweis sollte der erfindungsgemäß eingesetzte Johanniskrautextrakt kein nachweisbares Hypericin enthalten.

Es wurde im Rahmen der vorliegenden Erfindung überraschenderweise gefunden, daß die Kombination der beiden Extrakte einen synergistischen Effekt zeigt, der über eine bloße Addition der jeweiligen Wirksamkeiten deutlich hinausgeht.

Außerdem ist das Hyperforin per se relativ instabil. Überraschenderweise wurde auch gefunden, daß die erfindungsgemäß eingesetzten Extrakte aus Flechten zu einer deutlichen Stabilisierung von Hyperforin beitragen.

Die Kombination des Hypericum-Extraktes mit dem Usnea-Extrakt steigert die antiinflammatorische Wirkung der Einzelkomponenten um ca. das Zehnfache. Damit stellt die Kombination eines auf Hyperforin standardisierten Hypericum-Extraktes und eines auf Usninsäure standardisierten Usnea-Extraktes insbesondere in einer wässrigen Gelgrundlage ein besonders gut wirksames Präparat zur Behandlung von Akne und unreiner Haut dar.

Die Kombination von Hypericum-Extrakt und Usnea-Extrakt kann auch vorteilhaft in andere fettfreie Gelgrundlagen, die dem Fachmann allgemein bekannt sind, eingearbeitet werden. Wegen der Lipophilie der Komponenten ist aber auch die Einarbeitung der Komponenten in Lipidphasen möglich, z.B. in Form von Lipogelen, Cremes, Lotionen und Salben.

Die Kombination der Extrakte eignet sich nicht nur zur Behandlung mikrobieller Hauterkrankungen, wie Akne und unreine Haut. Wegen der davon unabhängigen ausgeprägten antientzündlichen Wirkung ist die Kombination der Extrakte auch hervorragend geeignet zur Behandlung entzündlicher Hauterkrankungen wie Ekzeme, Lichen ruber und Psoriasis, insbesondere wenn das Gesicht und der behaarte Kopf betroffen sind.

Die erfindungsgemäßen pharmazeutischen Zusammensetzungen enthalten bevorzugt 0,11 - 10 Gew.-% eines Extraktes aus Flechten und 0,11 - 10 Gew.-% eines Extraktes aus Johanniskraut und besonders bevorzugt 0,5 - 5 Gew.-% eines Extraktes aus Flechten und 0,5 - 5 Gew.-% eines Extraktes aus Johanniskraut.

Bei dem Extrakt aus Flechten handelt es sich bevorzugt um einen CO₂-Extrakt aus Bartflechten, der 3 - 6 Gew.-% Usninsäure enthält sowie Ester (angkettiger Fettsäuren mit mehrwertigen Alkoholen.

Die andere Komponente der erfindungsgemäßen Zusammensetzung ist der Extrakt aus Johanniskraut. Bevorzugt handelt es sich hierbei um einen CO₂-Extrakt aus Johanniskraut, der 5 - 15 Gew.-% Hyperforin enthält.

In einer bevorzugten Ausführungsform liegt die Konzentration des Hypericum-Extraktes bei 3 % und die des Usnea-Extraktes bei 2 %. Die bevorzugt eingesetzten Extrakte werden durch Hochdruckextraktion mit flüssigem Kohlendioxid hergestellt, wobei das Extraktverhältnis bevorzugt 3:1 bis 10:1 beträgt. Ebenso können mit anderen organischen Lösungsmitteln hergestellte Extrakte verwendet werden. Als Lösungsmittel kommen in Frage Ammoniak, Methanol, Buthanol und Hexan. Mit Methanol hergestellte Extrakte werden vorzugsweise als Trockenextrakte mit einem definierten Gehalt an Hyperforin bzw. Usninsäure eingesetzt. Alternativ können auch aufkonzentriertes oder reines Hyperforin oder stabile Lagerungsformen von Hyperforin, wie Hyperforinsalze, in einer flüssigen Usnea-Zubereitung aufgelöst werden. Die Einzelextrakte können auch nacheinander in eine Gelgrundlage eingearbeitet werden, wobei darauf zu achten ist, dass immer zuerst der Usnea-Extrakt eingearbeitet wird.

Im übrigen enthält die pharmazeutische Zusammensetzung neben den beiden pharmakologisch wirksamen Bestandteilen Zusatzstoffe, die in Abhängigkeit von der gewählten pharmazeutischen Formulierung zugegeben werden. Neben den Grundstoffen für Gels, Salben, Lotionen, Tinkturen etc. können die pharmazeutischen Zusammensetzungen gegebenenfalls Konservierungsstoffe, Antioxidationsmittel, Geruchsstoffe, Färbemittel und ähnliches enthalten. Es ist selbstverständlich, daß sich die einzelnen Komponenten auf 100 Gew.-% aufaddieren müssen.

In bevorzugter Ausführungsform werden die erfindungsgemäßen pharmazeutischen Zusammensetzungen als topische Formulierungsformen zur Verfügung gestellt. Topische Formulierungsformen werden vor allem bei der lokalen Behandlung von Erkrankungen der Haut und seltener bei systemischen Erkrankungen angewendet.

Dem Pharmazeuten ist eine Vielzahl von topischen Darreichungsformen bekannt. Gängige Ausführungsformen einer topischen Formulierung für dermatologische Erkrankungen sind Salben. Hierbei wird der pharmakologische Wirkstoff in einer halbfesten Salbengrundlage gelöst oder suspendiert. Üblicherweise handelt es sich hierbei um hydrophobe, streichfähige Cremen, die die Verdunstung von Wasser aus der Haut verhindern. Gerade bei der Behandlung von Akne sind aber Salben nicht bevorzugt, weil die Salbengrundlage das Auftreten von Hautunreinheiten begünstigen kann. Bei anderen Erkrankungen aber, beispielsweise Schuppenflechte, kann es durchaus vorteilhaft sein, die erfindungsgemäße pharmazeutische Zusammensetzung in eine Salbengrundlage einzuarbeiten. Die Salbengrundlage wird häufig durch flüssige, halbfeste und sogar feste Kohlenwasserstoffe gebildet, die üblicherweise aus Erdöl gewonnen werden. Es können aber auch natürliche Wachse (beispielsweise Bienenwachs) oder synthetische Wachse wie Cetylesterwachse eingesetzt werden. Auch organische Öle, insbesondere Olivenöl oder Baumwollsamenöl können in die Salben eingearbeitet werden. Durch die Mischung der einzelnen Bestandteile kann die Konsistenz der fertigen Salbe beeinflußt werden.

In einer weiteren Ausführungsform können die erfindungsgemäßen pharmazeutischen Zusammensetzungen in Cremes eingearbeitet werden. Hierbei handelt es sich um halbfeste Emulsionen, die flüssiger sind als Salben und leichter auf der Haut verteilbar sind. Die Grundbestandteile der Cremen können kleinere Mengen an Wasser aufnehmen und zu Emulsionen verarbeitet werden. Derartige Cremen sind in der Regel wasserabwaschbar und verstopfen die Poren der Haut nicht. Sie sind nicht fettig und ergeben beim Auftragen auf die Haut ein akzeptables Erscheinungsbild. Üblicherweise bestehen Cremen aus zwei Phasen, nämlich einer ölartigen internen Phase, die bevorzugt aus Kohlenwasserstoffen und hochmolekularen Alkoholen oder Fettsäuren besteht. Weiterhin enthalten die Cremen eine wäßrige Phase, die häufig Konservierungsstoffe, Feuchhaltemittel und Puffermittel enthält. Schließlich weisen die Cremen üblicherweise einen oder mehrere Emulgatoren auf. Häufig werden als Emulgatoren anionische, oberflächenaktive Mittel wie Natriumlaurylsulphat oder Triethanolaminstearat sowie nichtionische Tenside wie beispielsweise Ethylenoxidderivate verwendet. Kationische Tenside wie quaternäre Ammoniumsalze werden weniger bevorzugt, da sie häufig zu Hautirritationen führen können.

Um die pharmazeutischen Wirkstoffe besser lösen zu können, können die Cremes flüssige und wachsartige Komponenten wie beispielsweise Polyethylenglykol enthalten.

In einer bevorzugten Ausführungsform werden die erfindungsgemäßen pharmazeutischen Zusammensetzungen in wäßrige Gele eingearbeitet. Bei den wäßrigen Gelen wird eine flüssige, wässerige interne Phase in einer dreidimensionalen Matrix immobilisiert. Das System wird durch kolloidale Dispersionen von kleinen anorganischen oder großen organischen Molekülen in einem wäßrigen Medium bereitgestellt. Als gelbildende Stoffe werden bevorzugt nichtionische oder anionische Cellulosederivate wie beispielsweise Natriumcarboxymethylcellulose, saure Carboxyvinylpolymere oder andere hydrophile Kolloide wie Magnesiumaluminiumsilikat, Natriumalginat oder Tragant verwendet.

Bevorzugt können die erfindungsgemäßen pharmazeutischen Zusammensetzungen auch als Lotionen oder Lösungen eingesetzt werden, wobei Propylenglykol bevorzugt als Emulgator verwendet werden kann. Durch Verwendung von 5-10% Propylenglykol gelingt es, die lipophilen Extrakte in das Gel zu emulgieren.

In einer anderen Ausführungsform können die erfindungsgemäßen pharmazeutischen Zusammensetzungen auch als Puder Einsatz finden. Gerade bei der Anwendung auf fetter Haut kann es vorteilhaft sein, die pharmazeutische Zusammensetzung als Puder zu formulieren, wobei derartige Puder häufig auf anorganischer Grundlage hergestellt werden. Als Pudergrundlagen werden bevorzugt pulverige, saugfähige und gut deckende, an der Haut haftende ungiftige Stoffe verwendet. Bevorzugt eingesetzt wird Siliciumdioxid, gefällte Kreide, Magnesiumcarbonat, Zinkoxid sowie Talcum und Mischungen dieser Grundstoffe. Bei der pharmazeutischen Formulierung ist darauf zu achten, daß die Extrakte aus Flechten bzw. Johanniskraut in einer Form vorliegen müssen, daß sie von den Pudergrundlagen aufgesaugt bzw. adsorbiert werden können. Besonders bevorzugt eingesetzt werden hierfür deshalb Trockenextrakte nach Vorextraktion mit Methanol, mit definiertem Gehalt an Usninsäure und Hyperforin.

Schließlich können die erfindungsgemäßen pharmazeutischen Zusammensetzungen auch in Form eines wäßrigen Gels bereitgestellt werden, das durch Zugabe geeigneter Stoffe eine feste Struktur erhält, so daß bevorzugt transparente Stifte bereitgestellt werden, die die pharmazeutische Zusammensetzung enthalten und zum Abdecken von speziellen Hautunreinheiten eingesetzt werden können. Derartige Stifte enthalten bevorzugt mehrwertige aliphatische Alkohole mit bevorzugt 2 - 6 Kohlenstoffatomen wie Ethylenglykol, Propylenglykol, Trimethylenglykol, Glycerin und Mischungen dieser Verbindungen.

Zur Versteifung des wäßrigen Gels können auch gesättigte oder ungesättigte höhere Fettsäuren mit bevorzugt 14 bis 22 Kohlenstoffatomen eingesetzt werden. Beispiele hierfür sind Myristinsäure, Palmitinsäure, Stearinsäure, Oleinsäure, Linolensäure oder Mischungen dieser Fettsäuren.

Bevorzugt bei den galenischen Formulierungen ist die Auflösung eines auf Hyperforin standardisierten Hypericum-Extraktes in einem Usnea-Extrakt im o.g. Verhältnis und die anschließende Einarbeitung des Extraktgemisches in eine Nanoemulsion. Diese Nanoemulsion lässt sich vorteilhaft in eine wässrige Gelgrundlage einarbeiten.

Eine bevorzugte Gelgrundlage enthält Carbomer 50.0000, 2-Propanol, Propylenglykol und Wasser (siehe Rezepturbeispiel 1). Die Extraktkombination kann auch vorteilhaft eingearbeitet werden in andere wässrige Zubereitungsformen wie Lotionen, Sprays, Gesichtswässer und Kopfhautemulsionen. Eine besonders bevorzugte wässrige Lösung enthält den kombinierten Extrakt in einer Essenz mit Octyldodecanol, 2-Propanol, Propylenglykol und Wasser.

Für bestimmte Formulierungen kann es vorteilhaft sein, Zinksulfat-Heptahydrat in einer Konzentration von 0,5-1,5 %, bevorzugt 1,0 % zuzusetzen.

Über die Einarbeitung der Kombination eines auf Hyperforin standardisierten Hypericum-Extraktes und eines auf Usninsäure standardisierten Usnea-Extraktes in wässrige Trägersysteme hinaus eignet sich die Kombination der Extrakte also auch zur Einarbeitung in Cremes, Waschlotionen, Lipogele und Salben, deren prinzipielle Zusammensetzungen dem Fachmann allgemein bekannt sind.

Eine alternative Anwendungsform ist die Kombination der Extrakte zur innerlichen Anwendung in Form von Kapseln. Hierzu werden die lipophilen Extrakte in geeigneter Konzentration in ein hochwertiges Pflanzenöl eingearbeitet und unter Licht- und Sauerstoffschutz in opake Hartgelatinekapseln eingearbeitet. Ebenso geeignet ist die Einarbeitung der Extrakte in Form einer Mikroemulsion. Die Darreichung der Extrakte in Kapseln eignet sich zur Behandlung von Hauterkrankungen, die durch mikrobielle Infektion und Entzündung gekennzeichnet. Die Kapseln eignen sich insbesondere zur inneren Intensivbehandlung besonders hartnäckiger Erkrankungen des seborrhoischen Formenkreises, zum Beispiel Akne und Rosacea.

Die Formlierung der erfindungsgemäßen Wirkstoffkombination in Form von oral verabreichbaren Kapseln kann gerade bei besonders hartnäckigen Erkrankungen des seborrhoischen Formenkreises von Vorteil sein. Die Ausgestaltung einer geeigneten Kapsel ist dem Fachmann durchaus bekannt. So kann die Kapsel beispielsweise aus Gelatine bestehen. Derartige Kapseln werden im Magen aufgelöst und setzen die Wirkstoffkombination frei. Alternativ können die Hartgelatinekapseln auch mit einem magensaftresistenten Lack überzogen sein, so daß die Freisetzung des Wirkstoffes erst im Dünndarm erfolgt.

Mit den pharmazeutischen Zusammensetzungen der vorliegenden Erfindung wird ein Produkt auf natürlicher Basis bereitgestellt, das gegen die beim seborrhoischen Formenkreis relevanten Keime hoch wirksam ist und davon unabhängig zusätzlich entzündungshemmende Wirkung aufweist. Der verwendete Usnea-Extrakt wird nicht anderweitig als Antibiotikum eingesetzt und ist bevorzugt frei von Emulgatoren und Konservierungsmitteln. Aufgrund ihrer Lipophilie penetrieren die Wirkstoffe besonders gut in die Talgdrüsen, wo Sie Ihre antimikrobielle Wirkung entfalten. Die entzündungshemmende Wirkung wurde im Modell des UV-Erythemtests bei gesunden Probanden nachgewiesen.

Präparate mit Usneaextrakt liefern somit eine Basis zur Behandlung von Akne und ähnlichen Hauterkrankungen auf rein natürlicher Grundlage. Im verwendeten Usneaextrakt kommt gleichzeitig sowohl eine ausgezeichnete Wirkung gegen Aknebakterien, als auch gegen Pityrosporon-Hefepilze zur Geltung. Der Extrakt wirkt enzündungshemmend, ohne die Haut zu reizen. Durch Einarbeitung in ein wässriges Gel mit Propylenglykol gelingt es, den lipophilen Extrakt wasserlöslich zu machen. Da der Extrakt ein wirksames Antioxidans ist, können vorteilhaft zusätzliche oxidationsempfindliche Substanzen eingearbeitet und stabilisiert werden. Besonders vorteilhaft ist die Kombination des Usneaextraktes mit einem Hypericum CO₂-Extrakt, da hierdurch die Oxidation des Hypericum-Extraktes und der Abbau von Hyperforin verhindert, und seine entzündungshemmende Wirkung potenziert wird.

Figur 1 stellt in der Tabelle 1 die Wirksamkeit des erfindungsgemäß verwendeten Usnea-Extrakts gegenüber Aknebakterien (Propionibacterium acnes) dar.

Figur 2 stellt in der Tabelle 2 die Wirksamkeit des erfindungsgemäß verwendeten Usnea-Extrakts gegenüber Pityrosporon-Hefepilzen dar. Die Abkürzung "KBE" bedeutet "koloniebildende Einheit".

Figur 3 zeigt die stabilisierende Wirkung des Usnea-Extrakts auf den Extrakt aus Johanniskraut. In der Tabelle 3 ist deutlich zu sehen, daß der Anteil von Hyperforin fast doppelt so hoch ist, wenn Flechtenextrakt zugesetzt wurde. Diese zeitliche Stabilisierung ist für pharmazeutische Produkte sehr wichtig, da diese möglichst lange ohne Abbau der Wirkstoffe haltbar sein sollen.

Figur 4 zeigt die potenzierende Wirkung einer Kombination von Flechtenextrakt zusammen mit Johanniskrautextrakt im Modell des Lymphozyten-Proliferationstests

Figur 5 zeigt den entzündungshemmenden Effekt eines Usnea-Extrakts, der in einer Konzentration von 5 % in einem wässrigen Gel auf die Haut der Probanden aufgebracht wurde. Eine durch UVB-Strahlen hervorgerufene Entzündung kann durch die erfindungsgemäße pharmazeutische Zusammensetzung behandelt werden. Die erfindungsgemäßen pharmazeutischen Zusammensetzungen eignen sich daher auch zur Behandlung von durch übermäßige Sonnenbestrahlung hervorgerufene Entzündungen (Sonnenbrand).

### Herstellungsbeispiel 1: wässriges Gel mit Propylenglykol

3% Hypericum-Extrakt
2% Usnea barbata-Extrakt
Gelgrundlage ad 100,0

| Gelgrundlage | |
|---|---|
| Carbomer 50.000 | 0,5 |
| 2-Propanol | 5,0 |
| Propylenglykol | 20,0 |
| Natriumhydroxid | 0,12 |
| Gereinigtes Wasser | ad 100,0 |

### Herstellungsbeispiel 2: Gesichtswasser

3% Hypericum-Extrakt
2% Usnea barbata-Extrakt
1 % Zinksulfat-Heptahydrat
Alkoholische Essenz ad 100,0

| Alkoholische Essenz: | |
|---|---|
| Octyldodecanol | 18,0 |
| 2-Propanol | 58,0 |
| Propylenglykol | 10,0 |
| Gereinigtes Wasser | ad 100,0 |

### Eingesetzte Extrakte:

Johanniskrautextrakt: "St. John's wort CO₂to extract", Firma FLAVEX Usnea-Extrakt :"Usnea lichen CO₂to extract water soluble", Firma FLAVEX

### Beispiel 1. Antimikrobielle Wirkung des Bartflechten CO₂-Extraktes:

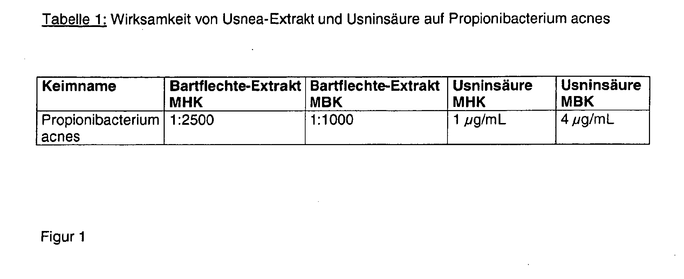

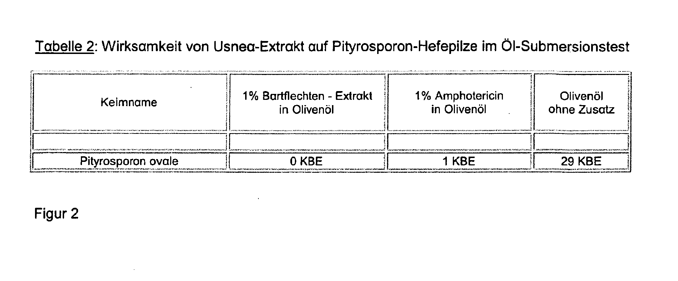

Der Bartflechten CO₂-Extrakt (Usnea barbata L.) wurde in den Konzentrationen 1:10, 1:50, 1:100, 1:500 und 1:1000 im Agardilutionstest bei einer Reihe von aeroben und anaeroben Bakterien geprüft. Die Reinsubstanz Usninsäure wurde in Konzentrationen von 128/ 64/ 32/16/ 8/ 4/ 2/ 1/ 0.5/ 0.25 und 0.125 µg/ mL getestet. Die MHK (Minimale Hemmkonzentration) ist definiert als > 90% Hemmung des Bakterienwachstums nach 24h. Die MBK (Minimale Bakterizide Konzentration) ist definiert als > 90% Hemmung des Wachstums bei 48h. Es zeigte sich überraschend, dass Propionibacterium acnes hochemfindlich auf den Usneaextrakt und auf Usninsäure reagiert (Tabelle 1, Figur 1).
Ausserdem wurde die Wirksamkeit gegen Pityrosporon von 1 % Usneaextrakt in Olivenöl im Vergleich zu 1 % Amphotericin untersucht. Hierzu wurden Schüppchen von Patienten mit seborrhoischem Ekzem auf einen Sabouraud-Agar aufgebracht und mit dem Öl überschichtet. Nach 4 Tagen Inkubation im Brutschrank wurde die Anzahl der koloniebildenden Einheiten ausgezählt. Das Wachstum der Pityrosporon-Hefepilze wurde vollständig durch den Usneaextrakt und Amphotericin, nicht aber durch Olivenöl alleine gehemmt (Tabelle 2, Figur 2).

### Beispiel 2. Potenzierung der antiproliferativen Wirkung durch Kombination von Hypericumextrakt und Usneaextrakt:

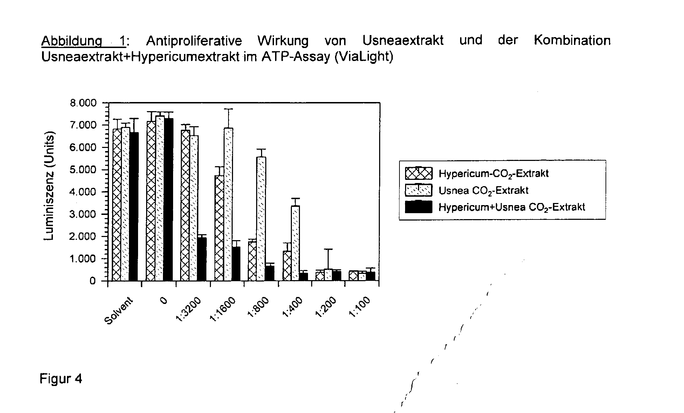

Eine Hemmung der Proliferation aktivierter Lymphozyten kann Ausdruck einer entzündungshemmenden Wirkung von Substanzen sein. Deshalb wurde die proliferationshemmende Wirkung von Usneaextrakt untersucht. Ausserdem wurde untersucht, ob Usneaextrakt die bekannte proliferationshemmende Wirkung eines Johanniskraut (Hypericum)-CO₂-Extraktes verstärken kann. Hierzu wurden Lymphozyten des peripheren Blutes (PBMC) über Ficoll-Dichtegradienten isoliert und in einer Zellzahl von 100.000/mL in Mikrotiterplatten ausgesät. Danach wurden die Zellen mit 1 µg/mL PHA (Phytohämagglutinin) stimuliert und mit oder ohne Zusatz von Pflanzenextrakten bzw. Lösungsmittelkontrolle für 24 h inkubiert. Folgende Stammlösungen wurden hergestellt: 25% Usneaextrakt in 70% EtOH, 25% Hypericumextrakt in EtOH, 12,5% Usneaextrakt+12,5% Hypericumextrakt in EtOH. Alle Stammlösungen wurden in einer Verdünnungsreihe von 1:100 bis 1:3200 eingesetzt. Danach wurde die Zellproliferation durch Messung des ATP-Gehaltes (ViaLight) bestimmt. Die halbe Hemmkonzentration (IC50) von Usneaextrakt tritt bereits bei einer Verdünnung von 1:400 auf. Es zeigte sich überaschend, dass der Zusatz von Usneaextrakt zum Hypericumextrakt nicht nur additiv wirkt, sondern dessen antiproliferative Wirkung potenziert (Abbildung 1, Figur 4).

### Beispiel 3. Entzündungshemmende Wirkung im UV-Erythemtest:

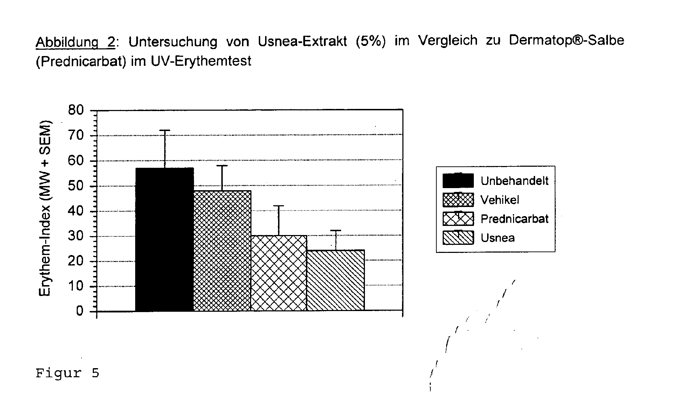

Bei 7 gesunden Probanden wurde eine Lichttreppe mit UVB durchgeführt. Anschliessend wurden Testfelder auf dem Rücken mit der 1,5 fachen minimalen Erythemdosis (MED) bestrahlt. Die Testsubstanzen wurde in Finn-Chambers für 24 Stunden aufgebracht. Das UV-induzierte Erythem wurde vor Bestrahlung und nach Einwirken der Substanzen photometrisch bestimmt. Es zeigte sich, dass 5% Usneaextrakt so gut wie das Cortisonpräparat Prednicarbat (Dermatop®) die UV-induzierte Entzündung hemmen kann (Abbildung 2, Figur 5).

### Beispiel 4. Anwendung von Usnea-Gel und Usnea/Hypericum-Gel bei Akne vulgaris:

Bei 3 Patienten kam es durch alleinige Anwendung von 3% Usneaextrakt in einer Gelgrundlage mit Propylenglykol zu einer Abheilung bzw. deutlichen Besserung der Akne-Effloreszenzen im Gesicht nach zwei Wochen Behandlung. Bei 2 Patienten kam es durch 3% Usnea+2% Hypericum-Gel in einer Woche zur Abheilung der Hautveränderungen im Gesicht.

### Beispiel 5. Stabilisierung von Hyperforin durch Zusatz von Usnea-Extrakt:

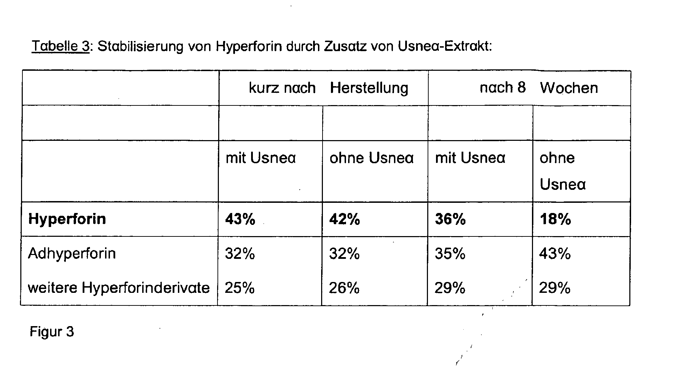

Es wurde überraschend gefunden, dass die Kombination eines auf 10 % Hyperforin standardisierten Johanniskrautextraktes mit einem auf 4 % Usninsäure standardisierten Usnea-Extrakt bei Einarbeitung in ein wässriges Gel mit Propylenglykol zu einer Zunahme der Stabilität von Hyperforin um 100 % führt (Tabelle 3, Figur 3).

## Patentansprüche

1. Pharmazeutische Zusammensetzung, **dadurch gekennzeichnet, daß** sie 0,01 - 20 Gew.-% eines Extraktes aus Flechten und 0,01 - 20 Gew.-% eines Extraktes aus Johanniskraut enthält.

2. Pharmazeutische Zusammensetzung nach Anspruch 1, **dadurch gekennzeichnet, daß** sie 0,11 - 10 Gew.-% eines Extraktes aus Flechten und 0,11 - 10 Gew.-% eines Extraktes aus Johanniskraut enthält.

3. Pharmazeutische Zusammensetzung nach Anspruch 1, **dadurch gekennzeichnet, daß** sie 0,5 - 5 Gew.-% eines Extraktes aus Flechten und 0,5 - 5 Gew.-% eines Extraktes aus Johanniskraut enthält.

4. Pharmazeutische Zusammensetzung nach einem der Ansprüche 1 bis 3, **dadurch gekennzeichnet, daß** es sich bei dem Extrakt aus Flechten um einen CO₂-Extrakt aus Bartflechten handelt.

5. Pharmazeutische Zusammensetzung nach Anspruch 4, **dadurch gekennzeichnet, daß** der CO₂-Extrakt aus Bartflechten 3 - 6 Gew.-% Usninsäure enthält.

6. Pharmazeutische Zusammensetzung nach einem der vorhergehenden Ansprüche, **dadurch gekennzeichnet, daß** es sich bei dem Extrakt aus Johanniskraut um einen CO₂-Extrakt aus Johanniskraut handelt.

7. Pharmazeutische Zusammensetzung nach Anspruch 6, **dadurch gekennzeichnet, daß** der CO₂-Extrakt aus Johanniskraut 5 - 15 Gew.-% Hyperforin enthält.

8. Pharmazeutische Zusammensetzung nach einem der vorhergehenden Ansprüche, **dadurch gekennzeichnet, daß** es sich um ein wäßriges Gel handelt.

9. Pharmazeutische Zusammensetzung nach einem der Ansprüche 1 bis 7, **dadurch gekennzeichnet, daß** es sich um eine Lotion handelt.

10. Pharmazeutische Zusammensetzung nach einem der Ansprüche 1 bis 7, **dadurch gekennzeichnet, daß** es sich um eine Salbe handelt.

11. Pharmazeutische Zusammensetzung nach einem der Ansprüche 1 bis 7, **dadurch gekennzeichnet, daß** es sich um ein versteiftes Gel handelt, das in Form eines Stiftes einsetzbar ist.

12. Pharmazeutische Zusammensetzung nach einem der Ansprüche 1 bis 7, **dadurch gekennzeichnet, daß** es sich um einen Puder handelt.

13. Verwendung einer Zusammensetzung nach einem der Ansprüche 1 bis 12 zur Herstellung eines Medikaments zur Behandlung einer Hauterkrankung des seborrhoischen Formenkreises.

14. Verwendung nach Anspruch 13, **dadurch gekennzeichnet, daß** es sich bei der Erkrankung um Akne handelt.

15. Verwendung nach einem der Ansprüche 13 oder 14, **dadurch gekennzeichnet, daß** es sich um eine topisch anwendbare Formulierung handelt.

16. Verwendung nach einem der Ansprüche 13 oder 14, **dadurch gekennzeichnet, daß** es sich um ein oral verabreichbares Medikament handelt.
